# EUROPÄISCHE PATENTSCHRIFT

(11) **EP 4 015 496 B1**
(45) Veröffentlichungstag und Bekanntmachung des Hinweises auf die Patenterteilung: **03.05.2023**
(21) Anmeldenummer: 20020615.9
(22) Anmeldetag: 15.12.2020
(51) Int. Cl.: C07C 29/151, C07C 31/04, B01J 8/02

(54) **VERFAHREN UND ANLAGE ZUM HERSTELLEN VON METHANOL AUS UNTERSTÖCHIOMETRISCHEM SYNTHESEGAS**
METHOD AND INSTALLATION FOR PRODUCING METHANOL FROM UNDER-STOICHIOMETRIC SYNTHESIS GAS
PROCÉDÉ ET INSTALLATION DE PRODUCTION DE MÉTHANOL À PARTIR DE GAZ DE SYNTHÈSE SUBSTOCHIOMÉTRIQUE

(43) Veröffentlichungstag der Anmeldung: 22.06.2022
(73) Patentinhaber: L'Air Liquide - Société Anonyme pour l'Etude et l'Exploitation des Procédés Georges Claude, 75321 Paris Cedex 07 (FR)
(72) Erfinder: Gronemann, Veronika, 60439 Frankfurt am Main (DE); Huder, Karin, 60439 Frankfurt am Main (DE)
(74) Vertreter: Dropsch, Holger

(56) Entgegenhaltungen:
- WO-A1-2006/018610
- US-A1- 2011 178 187
- US-A1- 2012 129 958
- US-A1- 2016 083 319

## Beschreibung

### Gebiet der Erfindung

Die Erfindung betrifft ein Verfahren zur Herstellung von Methanol durch heterogen-katalysierte Umsetzung von Wasserstoff und Kohlenoxide umfassendem Synthesegas, das bezüglich der für die Methanolsynthese optimalen Stöchiometrie einen Mangel an Wasserstoff aufweist, an festen, körnigen Katalysatoren zur Methanolsynthese, die in mehreren, hintereinandergeschalteten Festbettreaktoren angeordnet sind. Die Erfindung betrifft ferner eine Anlage zur Durchführung eines solchen Herstellungsverfahrens.

### Stand der Technik

Verfahren zur industriellen Herstellung von Methanol durch heterogen-katalytische Umsetzung von Synthesegas, also Gemischen von Wasserstoff und Kohlenoxiden, sind der Fachwelt seit langem bekannt. In Ullmann's Encyclopedia of Industrial Chemistry, Sixth Edition, 1998 Electronic Release, Kapitel "Methanol", Unterkapitel 5.2 "Synthesis" werden verschiedene Grundverfahren zur Herstellung von Methanol durch katalytische Umsetzung von Wasserstoff und Kohlenstoffoxide enthaltendem Synthesegas beschrieben, bei denen solche Reaktoren eingesetzt werden.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor (WCR) und dann einem gasgekühlten Reaktor (GCR) zugeführt wird, in welchen das Synthesegas jeweils an einem kupferbasierten Festbett-Katalysator zu Methanol umgesetzt wird, wobei in beiden Reaktoren ein Teilumsatz erfolgt und jeweils eine beträchtliche Menge nicht umgesetzten Synthesegases verbleibt. Das in dem Verfahren hergestellte Methanol wird von dem verbliebenen, zurückzuführenden Synthesegasabgetrennt, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten wie Methan innerhalb des Synthesekreislaufes anreichern. Diese Maßnahme wird auch in der deutschen Offenlegungsschrift DE 2934332 A1 und der europäischen Patentanmeldung EP 1016643 A1 gelehrt. Die WO 2006/018610 A1 offenbart ein zweistufiges Verfahren zur Herstellung von Methanol.

In der ersten, wassergekühlten Reaktorstufe wird üblicherweise der Hauptumsatz des Synthesegases (CO, CO₂, H₂) erzielt und der größte Teil der Reaktionswärme abgeführt, während in der zweiten, gasgekühlten Stufe bei milderen Bedingungen ein dennoch erheblicher Teil des Synthesegases umgesetzt wird.

In manchen mehrstufigen Anlagenkonfigurationen zur Methanolsynthese wird zusätzlich eine Zwischenkondensationsstufe zwischen den einzelnen Reaktionsstufen vorgesehen, um den Anteil der gebildeten Reaktionsprodukte (vorwiegend Methanol und Wasser) im Einsatzgas zur nachfolgenden Reaktionsstufe zu verringern. Dies bewirkt sowohl eine günstige Verschiebung der Gleichgewichtslage der Methanolbildungsreaktion in Richtung auf das Zielprodukt Methanol als auch eine Verringerung der Raumgeschwindigkeit bzw. Erhöhung der Verweilzeit im nachfolgenden Reaktor, die ebenfalls den Eduktumsatz weiter erhöht. Eine solche Anlagenkonfigurationen wird beispielsweise in der deutschen Patentschrift DE 10 2008 049 622 B4 gelehrt.

Bei dem wassergekühlten Reaktor (WCR) handelt es sich üblicherweise um einen Röhrenreaktor mit entsprechenden Rohrplatten, bei dem der Katalysator in die Rohre gefüllt wird, während die Kühlung mittels siedendem Wasser bzw. Dampferzeugung auf der Mantelseite um die Rohre erfolgt. Im gasgekühlten Reaktor (GCR) erfolgt die Kühlung mit dem Einsatzgas, welches durch die Rohre geführt wird und sich auf dem Weg zur ersten Reaktionsstufe (WCR) erwärmt, während der Katalysator um die Rohre gefüllt wird und die Reaktion auf der Mantelseite des GCR stattfindet. Die Reaktionsstufen sind hinsichtlich ihrer Nennweite mit großen bzw. sehr großen Rohrleitungen verbunden; je nach Anlagenkapazität sind Rohrdurchmesser von bis zu 1 m möglich. Dies ist vor allem auf die großen Gasmengen zurückzuführen, die zu der zweiten Stufe zurückgeführt (Recyclegas bzw. Rückführungsgas) und dem Frischgas oder Make-up-Gas, also frischem Synthesegas aus der Synthesegaserzeugung, zugemischt werden. Das sich ergebende Gasgemisch aus Rückführungsgas und Frischgas wird nach erfolgter Vorwärmung im GCR der ersten Reaktionsstufe (WCR) zugeführt. Die Rückführungsgasmenge (Recycle-Gas) ist üblicherweise deutlich größer als die Frischgasmenge und ist vom erzielten Umsatz in der Reaktorsektion abhängig. Das Rückführverhältnis RR (RR = R/F) aus Rückführungsgasmenge (R) zu Frischgasmenge (F) liegt oft über 2 und in einigen Fällen sogar über 3,5. Je niedriger der Umsatz von Synthesegas durch die Reaktorsektion per Durchgang ist, um so höher ist das erforderliche Rückführverhältnis RR, um eine hinreichende Ausbeute zu erzielen. Damit erhöht sich die umlaufende Gasmenge entsprechend, was die Belastung der Reaktoren erhöht und größere Rohrnennweiten der verbindenden Rohrleitungen bedarf und auch zu einem höherem Bedarf an Kompressionsenergie (höhere Durchflussmenge und Druckverlust) führt.

Üblicherweise werden in beiden Synthesereaktoren dieselben Methanolsynthesekatalysatoren auf Kupferbasis verwendet, die als feste, körnige Katalysatoren in Festbettreaktoren eingesetzt werden. Bei dem beschriebenen, zweistufigen WCR-GCR-Verfahren wird der wassergekühlte Reaktor typischerweise mit höherer Synthesegas-Eintrittstemperatur betrieben als ein wassergekühlter Reaktor in einem einstufigen Verfahren zur Methanolsynthese, um Dampf mit höherem Druck bereitstellen zu können. Ferner wird dieser Reaktor mit noch nicht abreagiertem Synthesegas beaufschlagt. Aufgrund der hohen Exothermie der Methanolsynthese ist daher eine sehr gute Temperaturkontrolle des Reaktors notwendig, um eine Überhitzung des Katalysators zu vermeiden, die wesentlich zu seiner vorzeitigen Desaktivierung beiträgt. In der DE-Offenlegungsschrift DE 102010008857 A1 wird daher vorgeschlagen, in den beiden Synthesereaktoren Katalysatoren mit unterschiedlicher Aktivität zu verwenden, wobei in dem Reaktor mit den drastischeren Reaktionsbedingungen ein Katalysator mit niedrigerer Aktivität eingesetzt werden soll, der eine geringere Desaktivierungsgeschwindigkeit und somit höhere Langzeitbeständigkeit aufweist.

Es existieren unterschiedliche Verfahren zur Herstellung von Wasserstoff und Kohlenoxide umfassendem Synthesegas als Einsatzgas für die Methanolsynthese, beispielsweise die Dampfreformierung, die autotherme Reformierung (ATR), Kombinationen beider (sog. Combined Reforming) und die nichtkatalytische Partialoxidation (POX). Als Ausgansstoffe dienen dabei Kohlenwasserstoffe wie Erdgas mit seiner Hauptkomponente Methan oder Naphtha. Die genannten Verfahren liefern unterschiedliche Verhältnisse der Produktkomponenten Kohlenmonoxid (CO) und Wasserstoff (H₂), wie anhand der folgenden Reaktionsgleichungen ersichtlich ist:

| | | |
|---|---|---|
| 2 CH₄ + O₂ | = 2 CO + 4 H₂ | (Partialoxidation) |
| 2 CH₄ + ½ O₂ + H₂O | = 2 CO + 5 H₂ | (autotherme Reformierung) |
| 2 CH₄ + 2 H₂O | = 2 CO + 6 H₂ | (reine Dampfreformierung) |

Da bei der Partialoxidation oder der autothermen Reformierung mit einem Überschuss an Kohlenwasserstoff bzw. Unterschuss an Sauerstoff gearbeitet wird, um die Totaloxidation des Kohlenwasserstoffs zu Kohlendioxid zu unterdrücken, wird oft ein Synthesegas erhalten, dass in Bezug auf seine Verwendung als Einsatzgas für die Methanolsynthese ein Wasserstoffdefizit aufweist. Diese erfordert gemäß folgender Reaktionsgleichung

2 H₂ + CO = CHsOH

ein H₂/CO-Verhältnis von mindestens 2, unter praktischen Synthesebedingungen oft sogar etwas größer als 2, beispielsweise 2,1. Dieses Verhältnis wird üblicherweise als Stöchiometriezahl SN der Methanolsynthese formuliert, die berücksichtigt, dass auch Kohlendioxid zu Methanol reagiert:

SN = ([H₂] - [CO₂]) / ([CO] + [CO₂]) ≥ 2 (z. B. 2,1)

Mittels Partialoxidation oder autothermer Reformierung erhaltene Synthesegase weisen dagegen oft eine Stöchiometriezahl von ≤ 1,9, gelegentlich sogar ≤ 1,7 auf. Zwar kann ein Synthesegas, das in Bezug auf die Methanolsynthese ein Wasserstoffdefizit aufweist, mittels der CO-Konvertierungsreaktion

CO + H₂O = CO₂ + H₂

hinsichtlich des geforderten Wasserstoffgehalts angepasst werden; nachteilig ist dabei jedoch die Tatsache, dass ein zusätzlicher Verfahrensschritt erforderlich ist, der zu erhöhten Investitions- und Betriebskosten und auch zu einer verstärkten Emission des klimaschädlichen Kohlendioxids führt. Eher wünschenswert wäre daher die Bereitstellung eines Einsatzgases mit der für die Methanolsynthese optimalen Stöchiometrie direkt durch das Synthesegaserzeugungsverfahren oder mittels einer Anpassung der Methanolsynthese selbst.

Eine solche Anpassung der Methanolsynthese kann darin bestehen, nicht umgesetzten Wasserstoff zurückzugewinnen und in die Synthese zurückzuführen. Wie oben ausgeführt, wird beim Durchgang des Synthesegases durch einen Methanolsynthesereaktor jeweils nur ein Teilumsatz zu Methanol erzielt und es verbleibt ein signifikanter Anteil nicht umgesetzten Synthesegases (sog. Restgas), das als Recyclegas zum Methanolsynthesereaktor zurückgeführt oder als Einsatzgas einem weiteren, stromabwärts angeordneten Methanolsynthesereaktor zugeführt wird. Dabei wird aus dem Restgasstrom vor seiner weiteren Verwendung ein Anteil als Spülstrom (Purgestrom) abgetrennt. Dieser Spülstrom enthält außer Inertkomponenten auch noch Wasserstoff und Kohlenoxide als nicht umgesetzte Synthesegasbestandteile. Er kann somit zur Verbesserung der Stöchiometriezahl des Synthesegases verwendet werden, wenn vor seiner Rückführung zu der Methanolsynthese eine Anreicherung seines Wasserstoffgehalts bzw. eine Abreicherung sowohl der Inertkomponenten als auch des Gehalts an Kohlenoxiden erfolgt. Dies kann mittels einer Wasserstoffrückgewinnungsvorrichtung erfolgen, die nach dem Prinzip der Druckwechseladsorption (PSA) und/oder nach dem Prinzip der Membrantrennung arbeitet und der der Spülstrom ganz oder teilweise zugeführt wird. Ein solches Konzept wird beispielsweise in den Patentveröffentlichungen US 7786180 B2 oder WO 2018/153625 A1 vorgeschlagen.

Bei der Einstellung eines optimierten Wasserstoffgehalts in den Reaktorfeedströmen ist ferner zu berücksichtigen, dass sich die Mengen und Zusammensetzungen der aus den Methanolsynthesereaktoren ausgeleiteten Produktgasströme fortlaufend verändern, was auf die zeitlich fortschreitende Desaktivierung der in den Reaktoren enthaltenen Katalysatoren zurückzuführen ist, die zu einem zeitlich abnehmendem, mittleren Umsatz in dem jeweiligen Reaktor führt. So erfolgt bei Verfahren mit mehreren hintereinander angeordneten Synthesereaktoren in der Regel zunächst eine fortschreitende Desaktivierung des Katalysators im strömungsmäßig ersten Reaktor, bevor zu einem späteren Zeitpunkt auch die Katalysatoraktivität des Katalysators im stromabwärts angeordneten, zweiten Reaktor abnimmt. Diesem Umstand wird unter anderem durch eine zeitliche Änderung des Rückführverhältnisses begegnet.

Sowohl die zeitlich fortschreitende Katalysatordesaktivierung als auch die zeitliche Änderung des Rückführverhältnisses machen es daher erforderlich, die Einstellung des Wasserstoffgehalts in den Reaktorfeedströmen zeitlich fortlaufend anzupassen. Hierzu bieten die oben genannten Veröffentlichungen aber keine Lösung an. Es besteht daher Bedarf an entsprechend modifizierten Verfahren und Anlagen, die diesen Erscheinungen Rechnung tragen und eine Methanolproduktion mit fortlaufend optimierter Zusammensetzung der Reaktorfeedströme gestatten.

### Beschreibung der Erfindung

Die Aufgabe der vorliegenden Erfindung besteht daher darin, ein Verfahren und eine Anlage anzugeben, das die beschriebenen Nachteile des Stands der Technik nicht aufweist und das es insbesondere ermöglicht, in einem mehrstufigen Verfahren bzw. einer mehrstufigen Anlage zur Methanolsynthese mit mehreren hintereinandergeschalteten Synthesereaktoren eine Methanolproduktion mit fortlaufend optimierter Zusammensetzung der Reaktorfeedströme zu gewährleisten.

Diese Aufgabe wird durch ein Verfahren mit den Merkmalen des Anspruchs 1 und durch eine Anlage mit den Merkmalen des Anspruchs 17 gelöst. Weitere Ausgestaltungen der Erfindung ergeben sich aus Unteransprüchen der jeweiligen Kategorie.

Unter Fluidverbindung zwischen zwei Bereichen der erfindungsgemäßen Vorrichtung wird dabei jegliche Art von Verbindung verstanden, die es ermöglicht, dass ein Fluid, beispielsweise ein Gasstrom, von dem einen zu dem anderen der beiden Bereiche strömen kann, unbeachtlich etwaiger zwischengeschalteter Bereiche oder Bauteile. Insbesondere wird unter einer direkten Fluidverbindung jegliche Art von Verbindung verstanden, die es ermöglicht, dass ein Fluid, beispielsweise ein Gasstrom, direkt von dem einen zu dem anderen der beiden Bereiche strömen kann, wobei keine weiteren Bereiche oder Bauteile zwischengeschaltet sind, mit Ausnahme reiner Transportvorgänge und der dazu benötigten Mittel, beispielsweise Rohrleitungen, Ventile, Pumpen, Verdichter, Vorratsbehälter. Ein Beispiel wäre eine Rohrleitung, die direkt dem einen zu dem anderen der beiden Bereiche führt.

Unter Synthesegaserzeugungsbedingungen und Methanolsynthesebedingungen sind die dem Fachmann an sich bekannten Verfahrensbedingungen, insbesondere von Temperatur, Druck und Verweilzeit, zu verstehen, wie sie detailliert im einschlägigen Schrifttum erörtert werden und bei denen mindestens ein Teilumsatz, bevorzugt allerdings technisch relevante Umsätze der jeweiligen Edukte zu den Produkten Synthesegas bzw. Methanol erfolgt. Entsprechend wird unter einem für die Methanolsynthese aktiven Katalysator ein Katalysator verstanden, der unter Methanolsynthesebedingungen eben solche Umsätze bewirkt. Notwendige Anpassungen dieser Bedingungen an die jeweiligen Betriebserfordernisse wird der Fachmann auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Für die Zwecke dieser Beschreibung ist Dampf synonym mit Wasserdampf zu verstehen, sofern es nicht im Einzelfall anders angegeben wird. Die Angabe "Wasser" bezieht sich dagegen auf Wasser im flüssigen Aggregatzustand, soweit nicht im Einzelfall anders angegeben.

Unter einem Kohlenstoff enthaltenden Einsatzstrom werden alle Einsatz-Stoffströme verstanden, die Kohlenstoff in elementarer oder chemisch gebundener, in der Regel organisch-chemisch gebundener Form enthalten, wobei es möglich ist, diesen enthaltenen Kohlenstoff unter Synthesegaserzeugungsbedingungen zu einem Kohlenoxide und Wasserstoff enthaltenden Synthesegas umzusetzen. Beispiele für häufig zur Synthesegaserzeugung eingesetzte, Kohlenstoff enthaltende Stoffströme sind Erdgas, Naphtha, Schweröl, schwersiedende Raffinerierückstände, Pyrolyseöle oder Pyrolyseteer aus Biomasse, Kohle mit unterschiedlicher Qualität, Partikelgröße und Inkohlungsgrad.

Unter einem weiteren Reinigungs-, Konditionierungs- oder Verarbeitungsschritt des Rohsynthesegases werden alle Maßnahmen und Verfahrensschritte verstanden, die zur Herstellung eines Reinsynthesegases, reinem Wasserstoff und/oder reinem Kohlenmonoxid aus dem Stand der Technik bekannt sind. Dazu zählen die CO-Konvertierung zur Erhöhung des Wasserstoffanteils im Synthesegas, die Abtrennung von Kohlendioxid mittels eines geeigneten Waschverfahrens wie beispielweise des Rectisol-Verfahrens oder die Wäsche mit aminhaltigen Waschmitteln, die kryogene Gaszerlegung zum Erzeugen von reinem Kohlenmonoxid, die Druckwechseladsorption (PSA) zum Erzeugen von Reinwasserstoff sowie physikalische Verfahrensschritte wie beispielsweise Abkühlen, Kondensieren, Abtrennen des Kondensats.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile sowie die entsprechenden Durchtrittsöffnungen in Behälterwänden in Betracht ziehen, die ihm aufgrund seines Fachwissens zur Durchführung dieses Verfahrensschrittes notwendig oder sinnvoll erscheinen.

Unter der katalytischen Aktivität oder der Katalysatoraktivität, insbesondere im Zusammenhang mit einer unterschiedlichen katalytischen Aktivität beim Vergleich zweier unterschiedlicher Katalysatoren, wird der erzielte Umsatzgrad pro Längeneinheit des Katalysatorbetts von Edukten zu Produkten verstanden. Die Aktivität wird beeinflusst von der chemischen Zusammensetzung, Dotierung, Vergiftung, verfügbaren Oberfläche etc. des Katalysatormaterials, aber auch durch die Geometrie der Katalysatorpartikel und texturellen Parametern des Katalysatorbetts, z. B. dessen Porosität oder Packungsdichte. Aufgrund der Exothermie der betrachteten Reaktionen korreliert eine hohe katalytische Aktivität mit einer hohen Wärmefreisetzung pro Längeneinheit des Katalysatorbetts. Ein weiteres Maß für die Katalysatoraktivität unter festgelegten Methanolsynthesebedingungen stellt der nach jeder Katalysatorstufe mittels Kondensation aufgefangene Mengenstrom an Flüssigprodukten dar, da die Reaktionsprodukte der Methanolsynthesereaktion gemäß der Umsatzgleichungen

CO (g) + 2 H₂ (g) = CHsOH (I)

CO₂ (g) + 3 H₂ (g) = CHsOH (I) + H₂O (I)

unter Umgebungsbedingungen flüssig sind. Demzufolge bezeichnet die Größe "Aktivitätsverlust" die zeitliche Abnahme der Katalysatoraktivität und des Edukt-Umsatzgrades bzw. der Methanol-Ausbeute als Maß für die Desaktivierung des Katalysators.

Unter der mittleren Katalysatoraktivität pro Katalysatorbett oder Reaktor wird die sowohl zeitlich (über einen Zeitabschnitt) als auch örtlich (über die Länge des Katalysatorbetts) gemittelte Katalysatoraktivität in dem betrachteten Katalysatorbett oder Reaktor verstanden.

Unter einem ersten bzw. zweiten Methanolsynthesereaktor werden nicht notwendigerweise Einzelreaktoren verstanden, sondern es können auch jeweils Gruppen von Einzelreaktoren von diesem Begriff umfasst werden, die ihrerseits wiederum ein oder mehrere Katalysatorzonen, also mit festem, körnigem Methanolsynthesekatalysator gefüllte Bereiche, beinhalten können. "Erster" bzw. "zweiter" Methanolsynthesereaktor soll dabei lediglich die Durchströmungsreihenfolge der betrachteten Reaktoren in Bezug auf den Feedstrom andeuten. Der erste bzw. zweite Methanolsynthesereaktor müssen nicht zwangsweise direkt aufeinander folgen, sondern es können weitere, nicht näher betrachtete Methanolsynthesereaktoren zwischengeschaltet sein.

Unter einem Katalysatorzyklus wird die Betriebsdauer einer Charge eines Methanolsynthesekatalysators verstanden, beginnend mit der Inbetriebnahme des Methanolsynthesebetriebs des mit der Charge frischen oder regenerierten Methanolsynthesekatalysators befüllten Methanolsynthesereaktors und endend mit der Außerbetriebnahme des Methanolsynthesebetriebs desselben Methanolsynthesereaktors zum Zwecke des Katalysatoraustauschs oder der Durchführung einer Katalysatorregenerierung.

Unter einem Auftrennen oder Aufteilen eines Stoffstroms ist im Zusammenhang mit der vorliegenden Erfindung ein Erzeugen mindestens zweier Teilströme aus einem Ausgangsstrom zu verstehen. Dabei werden beim Aufteilen mindestens zwei homogene, einphasige Teilströme aus dem Ausgangsstrom erzeugt, wobei beide Teilströme dieselbe stoffliche Zusammensetzung aufweisen wie der Ausgangsstrom. Dagegen erfolgt beim Auftrennen das Erzeugen der mindestens zwei Teilströme unter Anwendung eines Stofftrennschrittes aus dem Bereich der thermischen Verfahrenstechnik, also beispielsweise unter Anwendung eines Phasengleichgewichtes, z. B. bei der Verdampfung, Destillation, Kristallisation oder der Membrantrennung, so dass die mindestens zwei Teilströme in der Regel Zusammensetzungen und/oder Phasenanteile aufweisen, die sich untereinander sowie von dem Ausgangsstrom unterscheiden.

Unter einer Temperaturmessvorrichtung werden insbesondere entsprechende Messvorrichtungen verstanden, die es gestatten, die zeitliche Veränderung des axialen Temperaturprofils in einem Katalysatorbett zu verfolgen. Beispiele hierfür wären ein axial bewegliches Thermoelement oder ein axial im Katalysatorbett angeordnetes, fest angebrachtes Mehrpunkt-Thermoelement.

Die Angabe, dass ein Stoffstrom direkt einer spezifischen Verfahrensstufe oder einem spezifischen Anlagenteil zugeführt wird oder direkt in die jeweilige Verfahrensstufe oder den Anlagenteil eingeleitet wird, ist so zu verstehen, dass der Stoffstrom in diese Verfahrensstufe oder diesen Anlagenteil eingeleitet wird, ohne dass zuvor ein Durchleiten durch andere Verfahrensstufen oder Anlagenteile erfolgte, mit Ausnahme reiner Transportvorgänge und der dazu benötigten Mittel, beispielsweise Rohrleitungen, Ventile, Pumpen, Verdichter, Vorratsbehälter.

Methanol ist eine der wichtigsten weltweit produzierten petrochemischen Produkte. Das Verfahren zur Herstellung von Methanol kann in drei Hauptabschnitte unterteilt werden: Synthesegaserzeugung (einschließlich Synthesegasbehandlung, falls erforderlich), Methanolsynthese und Methanol-Destillation.

Einige der modernen, effizienten Verfahren zur Herstellung von Synthesegas können zu einer unterstöchiometrischen Synthesegaszusammensetzung führen, also zu einem Synthesegas mit Wasserstoffdefizit und einer Stöchiometriezahl kleiner als 2. Um die Stöchiometriezahl auf die erforderliche Zahl von 2 oder höher zu erhöhen, wird erfindungsgemäß dem unterstöchiometrischen Synthesegas Wasserstoff, der aus dem Spülgas des Gaskreislaufs der Methanolsynthese über eine oder mehrere spezielle Wasserstoffrückgewinnungsvorrichtungen gewonnen wird, hinzugefügt, um das sog. Make-Up-Gas (im Rahmen dieser Offenbarung als Synthesegaseinsatzstrom bezeichnet) zu erhalten.

Das aus dem Kreislauf abgetrennte Spülgasanteil bleibt bei Verfahren gemäß des Stands der Technik üblicherweise zeitlich weitgehend konstant_{[DH1]}, jedoch steigt der Spülgas-Mengenstrom und somit der Mengenstrom des aus dem Spülgas abgetrennten Wasserstoffs mit abnehmender Katalysatoraktivität von Beginn eines Katalysatorzyklus (Start of Run, SOR) in Richtung auf das Ende des Katalysatorzyklus (End of Run, EOR) an. Da der spezifische Wasserstoffbedarf, der zur Einstellung der gewünschten Stöchiometriezahl des Synthesegases benötigt wird, zeitlich konstant ist, entsteht über die Lebensdauer des Katalysators ein Überschuss an Wasserstoff, der gemäß Stand der Technik dem Brenngassystem zugeführt wird und somit nur thermisch, nicht aber stofflich genutzt wird. Daher ist es das Ziel der vorliegenden Erfindung, einen maßgeschneiderten Wasserstoffstrom zur Einstellung der Stöchiometriezahl des Synthesegases über die Lebensdauer des Katalysators anzubieten. Dies wird erfindungsgemäß dadurch erreicht, dass in üblichen Anlagen zur Methanolsynthese mindestens zwei Synthesereaktoren mit jeweils einem oder mehreren Katalysatorbetten pro Reaktor hintereinandergeschaltet sind. Oft erfolgt zudem zwischen den einzelnen Synthesereaktoren eine Abtrennung des erzeugten Methanols durch Auskondensieren, wobei das dabei verbliebene Restgas dem nachfolgenden Synthesereaktor zugeführt oder zum ersten Synthesereaktor zurückgeführt wird. Aus diesen Restgasen kann daher jeweils ein Anteil als Spülgas aus dem Synthesekreislauf ausgeleitet und einer oder mehreren Wasserstoffrückgewinnungsvorrichtungen zugeführt werden. Dabei ist zu berücksichtigen, dass die Desaktivierung der in den Synthesereaktoren enthaltenen Katalysatoren zumeist in Strömungsrichtung voranschreitet, wobei diese Erscheinung sowohl auf ein einzelnes betrachtetes Katalysatorbett als auch auf mehrere, hintereinander angeordnete Katalysatorbetten beobachtet wird. Bei einer Syntheseanordnung mit zwei hintereinandergeschalteten werden daher Desaktivierungserscheinungen zuerst in dem strömungsmäßig ersten Synthesereaktor auftreten und erst nachfolgend im strömungsmäßig zweiten Synthesereaktor.

Der Umsatz in den einzelnen Synthesereaktoren bzw. Methanolreaktionsschritten ändert sich mit abnehmender Katalysatoraktivität über die Lebensdauer des Katalysators. Die Aktivität des Katalysators wird durch Prozessparameter, Katalysatoralterung sowie Vergiftung aktiver Katalysatorzentren beeinflusst. Daher variiert die Gaszusammensetzung der abgeschiedenen Gasphase, die den einzelnen Reaktionsstufen zugeführt wird, über die Lebensdauer des Katalysators ebenso wie die Menge des abgeschiedenen Spülgases, während der spezifische Wasserstoffbedarf zur Anpassung der Stöchiometriezahl des definierten Synthesegases konstant ist. Daher werden die Anteile der Spülgasströme, die nach jedem Methanol-Abtrennungsschritt aus der Gasphase abgetrennt und zu einer oder mehreren Wasserstoffrückgewinnungsvorrichtungen geführt werden, so eingestellt und zeitlich variiert, dass die erforderliche Wasserstoffmenge zur Einstellung der gewünschten Stöchiometriezahl des Synthesegases erreicht wird.

Der Vorteil der Erfindung besteht somit darin, dass während der meisten Zeitabschnitte der Betriebsdauer der Methanolsyntheseanlage ein maßgeschneiderter Wasserstoff-Mengenstrom zur Einstellung der Stöchiometriezahl bereitgestellt werden kann, wobei durch die erfindungsgemäße, zeitliche Variation der mindestens zwei zu der oder den Wasserstoffrückgewinnungsvorrichtungen geführten Spülströme auf zeitliche Aktivitätsveränderungen der Katalysatoren in den mehreren hintereinandergeschalteten Synthesereaktoren reagiert werden kann. So kann entsprechend ein höherer Wasserstoffgehalt bei SOR-Bedingungen mit niedrigem Spülgasstrom und ein niedrigerer Wasserstoffgehalt bei EOR-Bedingungen mit höherem Spülgasstrom realisiert werden.

### Besondere Ausgestaltungen der Erfindung

In einem zweiten Aspekt ist das erfindungsgemäße Verfahren ist dadurch gekennzeichnet, dass der an Wasserstoff angereicherte Zusatzstrom vor dem Einleiten in den ersten Methanolsynthesereaktor mit dem Synthesegaseinsatzstrom und/oder mit dem Recyclestrom vermischt wird. Auf diese Weise wird gewährleistet, dass dem Reaktoreingang des strömungsmäßig ersten Synthesereaktors ein homogenes Gasgemisch aufgegeben wird und es wird die Bildung von Inhomogenitäten, beispielsweise örtlichen Konzentrationssträhnen, vermieden.

In einem dritten Aspekt ist das erfindungsgemäße Verfahren ist dadurch gekennzeichnet, dass der erste Spülstrom und der zweite Spülstrom in eine gemeinsame Wasserstoffrückgewinnungsvorrichtung eingeleitet werden. Hierdurch kann die Wasserstoffrückgewinnung auf besonders ökonomische Weise erfolgen und es werden Investitions- und Betriebskosten und der Energieverbrauch gegenüber der Verwendung von zwei getrennten Wasserstoffrückgewinnungsvorrichtungen eingespart. Ferner führt die Verwendung einer gemeinsamen Wasserstoffrückgewinnungsvorrichtung zu einer homogeneren Beschaffenheit des zum ersten Synthesereaktor zurückgeführten, an Wasserstoff angereicherten Zusatzstroms.

In einem vierten Aspekt ist das erfindungsgemäße Verfahren ist dadurch gekennzeichnet, dass die Wasserstoffrückgewinnungsvorrichtung nach dem Prinzip der Druckwechseladsorption (PSA) und/oder nach dem Prinzip der Membrantrennung arbeitet. Beide Verfahren zur Wasserstoffrückgewinnung und ihre jeweiligen Verfahrensbedingungen sind dem Fachmann grundsätzlich bekannt. Auch die Kombination beider Verfahren ist möglich und kann gegenüber den Einzelverfahren Vorteile erbringen.

In einem fünften Aspekt ist das erfindungsgemäße Verfahren ist dadurch gekennzeichnet, dass das Verhältnis der Stoffmengenströme n1s/n2s des ersten Spülstroms n1s zum zweiten Spülstrom n2s zeitlich verändert wird. Auf diese Weise kann die Stöchiometriezahl für das zum ersten Synthesereaktor geführte Synthesegas jeweils auf den Zielwert von mindestens 2 eingestellt werden.

In einem sechsten Aspekt ist das erfindungsgemäße Verfahren ist dadurch gekennzeichnet, dass das Verhältnis der Stoffmengenströme n1s/n2s des ersten Spülstroms n1s zum zweiten Spülstrom n2s zeitlich verändert wird und/oder dass die Summe des ersten Spülstroms und des zweiten Spülstroms n1s + n2s zeitlich verändert wird. Auf diese Weise kann auf die sich zeitlich verändernde Katalysatoraktivität in den mindestens zwei hintereinandergeschalteten Synthesereaktoren reagiert und die Stöchiometriezahl für das zum ersten Synthesereaktor geführte Synthesegas jeweils auf den Zielwert von mindestens 2 eingestellt werden.

In einem siebten Aspekt ist das erfindungsgemäße Verfahren ist dadurch gekennzeichnet, dass das Verfahren während eines ersten Zeitabschnitts t1 mit einem Verhältnis der Stoffmengenströme (n1s/n2s)1 und während eines zweiten Zeitabschnitts t2 mit einem Verhältnis der Stoffmengenströme (n1s/n2s)2 durchgeführt wird, wobei die mittlere Katalysatoraktivität des Katalysators im ersten Methanolsynthesereaktor während des ersten Zeitabschnitts höher ist als während des zweiten Zeitabschnitts. Auf diese Weise kann auf die sich während der Zeitabschnitte t1 und t2 zeitlich verändernde Katalysatoraktivität in den mindestens zwei hintereinandergeschalteten Synthesereaktoren reagiert und die Stöchiometriezahl für das zum ersten Synthesereaktor geführte Synthesegas jeweils auf den Zielwert von mindestens 2 eingestellt werden, indem im ersten Zeitabschnitt t1 ein Verhältnis der Stoffmengenströme (n1s/n2s)1 und während eines zweiten Zeitabschnitts t2 ein Verhältnis der Stoffmengenströme (n1s/n2s)2 eingestellt wird. Die Aufteilung der Betriebsdauer der Syntheseanlage in zwei Zeitabschnitte ist dabei nur exemplarisch und nicht einschränkend zu verstehen. Es kann durchaus sinnvoll sein, weitere Zeitabschnitte t3, t4 usw. zu definieren, während derer weitere Stoffmengenstrom-Verhältnisse (n1s/n2s)3, (n1s/n2s)4 usw. eingestellt werden, um eine besonders kleinschrittige Einstellung der gewünschten Stöchiometriezahl zu ermöglichen. Dabei sollte aber der vergrößerte Regelaufwand in Bezug auf das verbesserte Einstellen der Stöchiometriezahl vertretbar bleiben.

In einem achten Aspekt ist das erfindungsgemäße Verfahren ist dadurch gekennzeichnet, dass das Verfahren während eines ersten Zeitabschnitts mit einem Verhältnis der Stoffmengenströme (n1s/n2s)1 und während eines ersten Zeitabschnitts mit einem Verhältnis der Stoffmengenströme (n1s/n2s)2 durchgeführt wird, wobei (n1s/n2s)1 größer ist als (n1s/n2s)2. Während des ersten Zeitabschnitts, beginnend mit der Inbetriebnahme des Verfahrens bzw. der Anlage, ist im ersten und im zweiten Methanolsynthesereaktor jeweils frischer Katalysator vorhanden. Daher wird der im ersten Reaktorfeedstrom enthaltene Wasserstoff weitgehend im ersten Methanolsynthesereaktor umgesetzt und es muss ein vergleichsweise großer Stoffmengenstrom des ersten Spülstroms zu der Wasserstoffrückgewinnungsvorrichtung geführt werden, um eine bestimmte Wasserstoffmenge zu gewinnen. Während des nachfolgenden zweiten Zeitabschnitts sind die in den Methanolsynthesereaktoren enthaltenen Katalysatoren dagegen bereits teilweise desaktiviert; das Reaktionsgeschehen verlagert sich daher zunehmend vom ersten in den stromabwärts angeordneten zweiten Methanolsynthesereaktor. Am Ausgang des ersten Methanolsynthesereaktors steht daher mehr nicht umgesetzter Wasserstoff zur Verfügung, so dass der zu der Wasserstoffrückgewinnungsvorrichtung geführte Stoffmengenstrom des ersten Spülstroms reduziert werden kann.

In einem neunten Aspekt ist das erfindungsgemäße Verfahren ist dadurch gekennzeichnet, dass der erste Zeitabschnitt t1 mit der Inbetriebnahme des Verfahrens beginnt und dass der zweite Zeitabschnitt t2 mit der Außerbetriebnahme des Verfahrens endet. Da im ersten und zweiten Methanolsynthesereaktor die Katalysatoraktivität bei Inbetriebnahme des Verfahrens hoch und vor der Außerbetriebnahme des Verfahrens wegen der mindestens teilweisen Desaktivierung niedriger ist, ergeben sich zwischen diesen beiden Zeitabschnitten deutliche Änderungen der Gaszusammensetzungen am Ausgang des ersten und zweiten Methanolsynthesereaktors, so dass eine Nachführung des Verhältnisses der Stoffmengenströme (n1s/n2s) sinnvoll ist.

In einem zehnten Aspekt ist das erfindungsgemäße Verfahren ist dadurch gekennzeichnet, dass mit dem Beginn des ersten Zeitabschnitts t1 der Katalysatorzyklus in allen Methanolsynthesereaktoren beginnt (SOR) und dass mit dem Ende des zweiten Zeitabschnitts t2 der Katalysatorzyklus in allen Methanolsynthesereaktoren endet (EOR). Die Vorteile entsprechen denen, die im Zusammenhang mit dem neunten Aspekt der Erfindung erörtert wurden.

In einem elften Aspekt ist das erfindungsgemäße Verfahren ist dadurch gekennzeichnet, dass das Verhältnis der Stoffmengenströme (n1s/n2s)1 größer als 1 ist und dass das Verhältnis der Stoffmengenströme (n1s/n2s)2 kleiner als 1 ist. Dies spiegelt die Erkenntnis wider, dass während des ersten Zeitabschnitts ein vergleichsweise großer Stoffmengenstrom des ersten Spülstroms zu der Wasserstoffrückgewinnungsvorrichtung geführt werden, um eine bestimmte Wasserstoffmenge zu gewinnen, wobei n1s größer ist als n2s. Während des zweiten Zeitabschnitts verlagert sich der Hauptteil der Reaktion in den zweiten Methanolsynthesereaktor, der erste Spülstrom kann reduziert werden und das Verhältnis (n1s/n2s)2 fällt unter 1.

In einem zwölften Aspekt ist das erfindungsgemäße Verfahren ist dadurch gekennzeichnet, dass das Verhältnis der Stoffmengenströme (n1s/n2s)1 zwischen 1 und 3, bevorzugt zwischen 2 und 2,9, meist bevorzugt zwischen 2,5 und 2,8 beträgt und dass das Verhältnis der Stoffmengenströme (n1s/n2s)2 zwischen 0 und 1, bevorzugt zwischen 0,3 und 0,9, meist bevorzugt zwischen 0,4 und 0,7 beträgt. Untersuchungen und Berechnungen haben ergeben, dass diese Wertebereiche besondere Vorteile erbringen und es insbesondere ermöglichen, eine Stöchiometriezahl von größer als 2 und ein günstiges Rückführverhältnis RR zu realisieren.

In einem dreizehnten Aspekt ist das erfindungsgemäße Verfahren ist dadurch gekennzeichnet, dass die zeitliche Abnahme des Mengenstroms des ersten Flüssigproduktstroms als Maß für das Abnehmen der Katalysatoraktivität des Katalysators im ersten Methanolsynthesereaktor dient. Auf diese Weise kann die Zwischenkondensation und Abscheidung des Methanolprodukts nach jedem Synthesereaktor vorteilhaft zu Aktivitätsbestimmung des Katalysators in diesem Reaktor genutzt werden, da der zeitliche Verlauf der Produktmenge pro Reaktor direkt über den Mengenstrom der aufgefangenen, kondensierten Produkte gemessen werden kann, so dass aufwändige Gasanalysen und Durchflussmessungen entfallen.

In einem vierzehnten Aspekt ist das erfindungsgemäße Verfahren ist dadurch gekennzeichnet, dass mittels einer Temperaturmessvorrichtung wiederholt während der Durchführung des Verfahrens axiale Temperaturprofile innerhalb des Katalysatorbetts im ersten Methanolsynthesereaktor gemessen werden und dass die zeitliche Veränderung des axialen Temperaturprofils als Maß für das Abnehmen der Katalysatoraktivität des Katalysators im ersten Methanolsynthesereaktor dient. Als Temperaturmessvorrichtungen können dabei Einpunkt- oder Mehrpunkt-Thermoelemente dienen, die jeweils parallel zur Längsachse des Katalysatorbetts und in dessen Innerem, bevorzugt in dessen Zentrum, angeordnet sind. Einpunkt-Thermoelemente werden dabei zumeist in einem axialen Führungsrohr in das Katalysatorbett eingebracht und sind in diesem verschiebbar, so dass durch stückweises Herausziehen im zeitlichen Turnus die Temperatur an bestimmten Positionen des Katalysatorbetts gemessen werden kann, so dass ein zu einem bestimmten Betriebszeitpunkt gehörendes axiales Temperaturprofil erhalten wird. Mehrpunkt-Thermoelemente sind fest in dem Katalysatorbett angeordnet und messen die Temperatur an verschiedenen axialen Positionen des Katalysatorbetts zu bestimmten Zeitpunkten, so dass auch auf diese Weise axiale Temperaturprofile im Katalysatorbett in Abhängigkeit von der Betriebszeit des Synthesereaktors erhalten werden können. Aufgrund der starken, exothermen Wärmetönung der Methanolsynthesereaktion gestatten es beide Möglichkeiten, Aussagen über den Fortgang des Reaktionsgeschehens und das zeitliche Fortschreiten der Katalysatordesaktivierung zu treffen.

In einem fünfzehnten Aspekt ist das erfindungsgemäße Verfahren ist dadurch gekennzeichnet, dass das Synthesegaserzeugungsverfahren eine nichtkatalytische Partialoxidation (POX) und/oder eine autotherme Reformierung (ATR) umfasst. Da bei der Partialoxidation oder der autothermen Reformierung mit einem Überschuss an Kohlenwasserstoff bzw. Unterschuss an Sauerstoff gearbeitet wird, um die Totaloxidation des Kohlenwasserstoffs zu Kohlendioxid zu unterdrücken, wird oft ein Synthesegas erhalten, dass in Bezug auf seine Verwendung als Einsatzgas für die Methanolsynthese ein Wasserstoffdefizit aufweist. Daher bietet das erfindungsgemäße Verfahren zur Methanolsynthese bei diesen Synthesegaserzeugungsverfahren besondere Vorteile und das jeweilige Wasserstoffdefizit kann hiermit wirkungsvoll ausgeglichen werden.

In einem sechzehnten Aspekt ist das erfindungsgemäße Verfahren ist dadurch gekennzeichnet, dass die Stöchiometriezahl des Synthesegaseinsatzstroms unter 2 beträgt und dass die Stöchiometriezahl des aus dem Synthesegaseinsatzstrom, dem Recyclestrom und dem Zusatzstrom erhaltenen Gasgemischs über 2 beträgt, bevor dieses in den ersten Methanolsynthesereaktor eingeleitet wird. Wie oben erläutert, werden Synthesegase mit einer Stöchiometriezahl unter 2 oft dann erhalten, wenn das Synthesegaserzeugungsverfahren eine nichtkatalytische Partialoxidation (POX) und/oder eine autotherme Reformierung (ATR) umfasst. Daher bietet das erfindungsgemäße Verfahren zur Methanolsynthese bei diesen Synthesegaserzeugungsverfahren besondere Vorteile und das jeweilige Wasserstoffdefizit kann hiermit wirkungsvoll ausgeglichen werden.

In einem weiteren Aspekt ist das erfindungsgemäße Verfahren dadurch gekennzeichnet, dass das Verfahren auch einen dritten Methanolsynthesereaktor umfasst, aus dessen Reaktorproduktstrom ein weiterer Restgasstrom gewonnen wird, der nicht umgesetzte Synthesegasbestandteile enthält, wobei aus dem weiteren Restgasstrom ein weiterer Spülstrom gewonnen wird, der einer Wasserstoffrückgewinnungsvorrichtung zugeführt wird. Hierdurch ist eine noch feinere Reaktion auf die fortschreitende Katalysatordesaktivierung in den hintereinandergeschalteten Synthesereaktoren möglich als bei der Anwesenheit von nur zwei Synthesereaktoren. Dagegen ist die Bereitstellung von mehr als drei Synthesereaktoren wegen der erhöhten Investitions- und Betriebskosten zumeist nicht sinnvoll.

In einem weiteren Aspekt ist das erfindungsgemäße Verfahren dadurch gekennzeichnet, dass der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird. Der gasgekühlte Reaktor erfüllt somit zwei Funktionen, nämlich als Synthesereaktor und als Wärmetauscher bzw. Feedvorwärmer für den WCR. Eine gute Temperaturkontrolle der Synthesereaktoren durch die erfindungsgemäße Steuerung des Reaktionsverlaufs auch bei zunehmender Katalysatordesaktivierung ist daher bei der Ausgestaltung gemäß WCR-GCR-Konzept besonders wichtig.

In einem weiteren Aspekt ist das erfindungsgemäße Verfahren dadurch gekennzeichnet, dass der zweite Methanolsynthesereaktor als gasgekühlter Reaktor (GCR) ausgestaltet ist, wobei der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird.

### Ausführungs- und Zahlenbeispiele

Weiterbildungen, Vorteile und Anwendungsmöglichkeiten der Erfindung ergeben sich auch aus der nachfolgenden Beschreibung von Ausführungs- und Zahlenbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigt die einzige Figur:

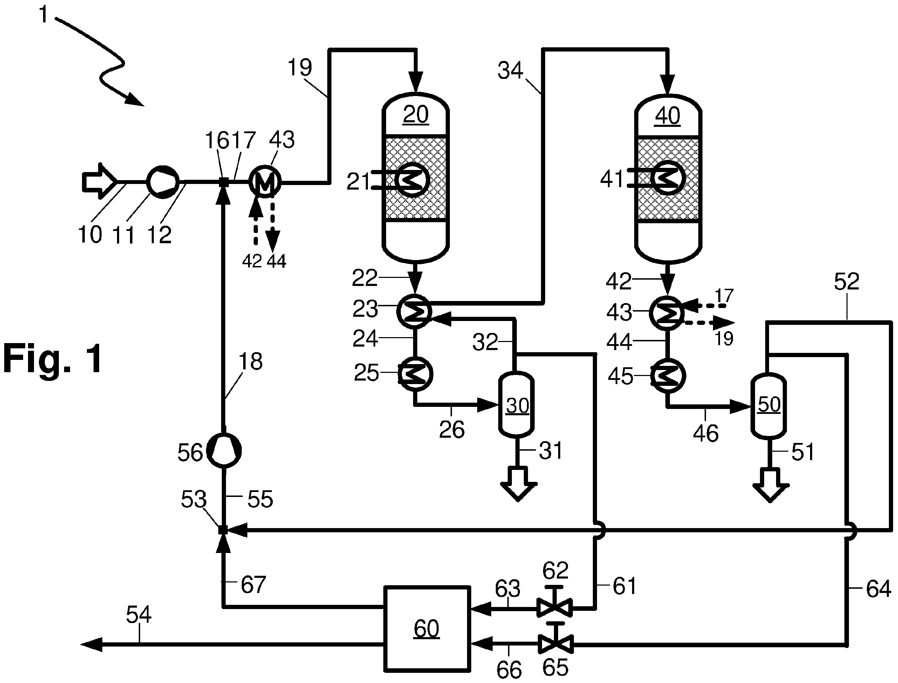

- Fig. 1: eine schematische Darstellung des Verfahrens bzw. der Anlage gemäß einer beispielhaften Ausführungsform der Erfindung.

In der in Fig. 1 dargestellten, beispielhaften Ausgestaltung eines Verfahrens 1 bzw. einer Anlage 1 gemäß der Erfindung, die zwei hintereinander geschaltete, wassergekühlte Synthesereaktoren 20, 40 für die Methanolsynthese umfasst, wird ein frischer Synthesegasstrom, enthaltend Wasserstoff, Kohlenmonoxid und Kohlendioxid und eine Stöchiometriezahl von kleiner als 2 aufweisend, aus einer nicht dargestellten Synthesegaserzeugungsanlage, beispielsweise einer POX-Anlage oder einer ATR-Anlage, über Leitung 10 eingeleitet, mittels Verdichter 11 auf Synthesedruck verdichtet und über Leitung 12 als Synthesegaseinsatzstrom (synonym Frischgas, Make-up-Gas) zu Mischvorrichtung 16 geleitet und in dieser mit einem Recyclestrom zusammengeführt, der über Leitung 18 herangeführt und ebenfalls in die Mischvorrichtung 16 eingeleitet wird. Die Mischvorrichtung 16 kann, wie auch die nachfolgend genannten Mischvorrichtungen, zum Beispiel als T-Rohrstück oder als statischer Mischer ausgestaltet sein. Das Verhältnis der Mengenströme, die mittels der Leitungen 18 (Recyclestrom) und 12 (Frischgas) zu der Mischvorrichtung 16 geführt werden, entspricht dem Rückführverhältnis RR.

Durch das Zusammenführen und Vermischen des Synthesegaseinsatzstroms mit dem Recyclestrom wird ein erster Reaktorfeedstrom erhalten, der über Leitung 17 zu Wärmetauscher 43 geführt wird und in diesem beispielsweise im indirekten Wärmetausch gegen den heißen Reaktorproduktstrom aus dem zweiten Synthesereaktor 40 (angedeutet durch die gestrichelten Leitungen 42, 44) auf die Reaktoreintrittstemperatur aufgeheizt wird. Er wird sodann über Leitung 19 in den ersten Methanolsynthesereaktor 20 eingeleitet.

Im ersten Methanolsynthesereaktor 20, der mindestens eine Katalysatorzone mit einem einen für die Methanolsynthese aktiven, festen, körnigen Katalysator enthält, erfolgt die Teilumsetzung des ersten Reaktorfeedstroms unter Methanolsynthesebedingungen. Im Ausführungsbeispiel der Fig. 1 sind beide Synthesereaktoren 20, 40 wassergekühlt; die jeweiligen in die Reaktoren integrierten Kühlvorrichtungen sind mittels Bezugszeichen 21, 41 angedeutet. Auch andere Wärmeintegrations- und Kühlkonzepte sind möglich (bildlich nicht gezeigt); so kann der erste Reaktorfeedstrom auch als Kühlgasstrom in einem der Reaktoren (GCR) genutzt werden, wie es das Verfahren gemäß der Patentschrift EP 0 790 226 B1 vorschlägt. Der andere Methanolsynthesereaktor wird dabei beispielsweise mit Kühlwasser gekühlt (WCR).

Aus dem ersten Synthesereaktor 20 wird über Leitung 22 ein heißer erster Reaktorproduktstrom ausgeleitet und zu Wärmetauscher 23 und sodann über Leitung 24 zu Kühler 25 geführt. In Wärmetauscher 23 erfolgt eine erste Abkühlung des heißen ersten Reaktorproduktstroms durch indirekten Wärmetausch gegen den aus der ersten Phasentrennvorrichtung 30 ausgeleiteten, abgekühlten Restgasstrom. Kühler 25 kann beispielsweise als Luftkühler oder als mit Kühlwasser betriebener Kühler ausgestaltet werden. Der unter seinen Taupunkt abgekühlte, erste Reaktorproduktstrom wird über Leitung 26 in eine erste Phasentrennvorrichtung 30 eingeleitet und in dieser in einen ersten Flüssigproduktstrom und in einen ersten Restgasstrom aufgetrennt. Der erste Flüssigproduktstrom, der im Wesentlichen Methanol und Wasser enthält, wird über Leitung 31 aus dem Verfahren bzw. aus der Anlage ausleitet und einer bildlich nicht dargestellten Rohmethanolaufarbeitung zugeführt, die beispielsweise einen oder bevorzugt mehrere Destillationsschritte umfasst. Der erste Restgasstrom, der noch nicht umgesetzte Synthesegasbestandteile sowie Inertgasbestandteile im Sinne der Methanolsynthese, beispielsweise Methan, enthält, wird über Leitung 32 zu Wärmetauscher 23 geführt und in diesem im indirekten Wärmetausch gegen den heißen ersten Reaktorproduktstrom aus dem Methanolsynthesereaktor 20 aufgeheizt. Anschließend wird der nunmehr aufgeheizte erste Restgasstrom über Leitung 34 zum zweiten Methanolsynthesereaktor 40 geführt und in diesen eingeleitet, der ebenfalls mindestens eine Katalysatorzone mit einem einen für die Methanolsynthese aktiven, festen, körnigen Katalysator enthält. Im zweiten Methanolsynthesereaktor erfolgt die Teilumsetzung des zweiten Reaktorfeedstroms unter Methanolsynthesebedingungen.

Aus dem zweiten Methanolsynthesereaktor 40 wird über Leitung 42 ein heißer zweiter Reaktorproduktstrom ausgeleitet und zu Wärmetauscher 43 und sodann über Leitung 44 zu Kühler 45 geführt. Im Wärmetauscher 43 erfolgt eine erste Abkühlung des heißen zweiten Reaktorproduktstroms, beispielsweise durch indirekten Wärmetausch gegen den über Leitung 17 herangeführten und über Leitung 19 abgeführten ersten Reaktorfeedstrom (beide Leitungen 17, 19 gestrichelt angedeutet). Kühler 45 kann ebenfalls beispielsweise als Luftkühler oder als mit Kühlwasser betriebener Kühler ausgestaltet werden. Der unter seinen Taupunkt abgekühlte, zweite Reaktorproduktstrom wird über Leitung 46 in eine zweite Phasentrennvorrichtung 50 eingeleitet und in dieser in einen zweiten Flüssigproduktstrom und in einen zweiten Restgasstrom aufgetrennt. Der zweite Flüssigproduktstrom, der wiederum im Wesentlichen Methanol und Wasser enthält, wird über Leitung 51 aus dem Verfahren bzw. aus der Anlage ausleitet und der bildlich nicht dargestellten Rohmethanolaufarbeitung zugeführt. Der zweite Restgasstrom, der noch nicht umgesetzte Synthesegasbestandteile enthält, wird als Recyclestrom über Leitung 52 zu Mischvorrichtung 53 geführt.

Erfindungsgemäß wird stromabwärts der ersten Phasentrennvorrichtung 30 aus Leitung 32 über Leitung 61 ein Teilstrom des ersten Restgasstroms als erster Spülstrom, und stromabwärts der zweiten Phasentrennvorrichtung 50 aus Leitung 52 über Leitung 64 ein Teilstrom des zweiten Restgasstroms als zweiter Spülstrom abgeführt. Das Verhältnis der über Leitungen 61 und 64 abgeführten, ersten und zweiten Spülströme und der Gesamtmengenstrom, der sich aus der Summe beider Spülströme ergibt, kann mittels der Regelventile 62 und 65 eingestellt werden. Der erste Spülstrom wird sodann über Leitung 63 und der zweite Spülstrom über Leitung 66 einer gemeinsamen Wasserstoffrückgewinnungsvorrichtung 60 zugeführt und in diese eingeleitet. Hierdurch kann die Wasserstoffrückgewinnung auf besonders ökonomische Weise erfolgen und es werden Investitions-und Betriebskosten und der Energieverbrauch gegenüber der Verwendung von zwei getrennten Wasserstoffrückgewinnungsvorrichtungen eingespart. Ferner führt die Verwendung einer gemeinsamen Wasserstoffrückgewinnungsvorrichtung zu einer homogeneren Beschaffenheit des zum ersten Synthesereaktor zurückgeführten, an Wasserstoff angereicherten Zusatzstroms.

Die Wasserstoffrückgewinnungsvorrichtung kann beispielsweise nach dem Prinzip der Druckwechseladsorption (PSA) und/oder nach dem Prinzip der Membrantrennung arbeiten. Beide Verfahren zur Wasserstoffrückgewinnung und ihre jeweiligen Verfahrensbedingungen sind dem Fachmann grundsätzlich bekannt. Auch die Kombination beider Verfahren ist möglich und kann gegenüber den Einzelverfahren Vorteile erbringen. Insbesondere bei Verwendung einer PSA-Anlage als Wasserstoffrückgewinnungsvorrichtung erbringt die Verwendung einer gemeinsamen Wasserstoffrückgewinnungsvorrichtung Vorteile hinsichtlich der Einsparung von Investitionskosten und der Reduzierung des benötigten Platzbedarfs.

In der Wasserstoffrückgewinnungsvorrichtung 60 erfolgt das Auftrennen des ersten Spülstroms und des zweiten Spülstroms in der in einen an Wasserstoff angereicherten Zusatzstrom, der über Leitung 67 ausgeitet wird, und in einen an Wasserstoff abgereicherten, dritten Spülstrom, der über Leitung 54 aus dem Verfahren ausgeleitet und einer bildlich nicht dargestellten Abgasentsorgung oder Abgasweiterverwertung zugeführt wird. Durch das Ausleiten des dritten Spülstroms aus dem Verfahren wird die Anreicherung von Inertgasen im Kreislauf der Methanolsynthese wirkungsvoll vermieden. Hierzu gehören im Sinne der Methanolsynthese nicht reaktive Gase, beispielsweise Edelgase oder Methan.

Der an Wasserstoff angereicherte, über Leitung 67 aus der Wasserstoffrückgewinnungsvorrichtung ausgeleitete Zusatzstrom wird mit dem über Leitung 52 herangeführten Recyclestrom in der Mischvorrichtung 53 zusammengeführt, bei der es sich beispielsweise um einen statischen Mischer oder ein einfaches T-Rohrstück handeln kann. Hierdurch wird ein an Wasserstoff angereicherter Recyclestrom erhalten, der über Leitung 55 zu einem Verdichter 56 geführt und in diesen eingeleitet wird. Der verdichtete, an Wasserstoff angereicherte Recyclestrom wird über Leitung 18 aus Verdichter 56 ausgeleitet und in Mischvorrichtung 16 eingeleitet, wodurch sich ein an Wasserstoff angereicherter, erster Reaktorfeedstrom ergibt, der nunmehr eine Stöchiometriezahl von größer als 2, beispielsweise 2,1, aufweist.

In weiteren, bildlich nicht dargestellten Beispielen ist es möglich, den an Wasserstoff angereicherten, über Leitung 67 aus der Wasserstoffrückgewinnungsvorrichtung ausgeleiteten Zusatzstrom zunächst mit dem Synthesegaseinsatzstrom oder mit dem Recyclestrom und dem Synthesegaseinsatzstrom zu vermischen, bevor das erhaltene Gasgemisch als an Wasserstoff angereicherter, erster Reaktorfeedstrom in den ersten Synthesereaktor eingeleitet wird.

Mit der der erfindungsgemäßen Rückgewinnung von Wasserstoff aus dem ersten und zweiten Spülstrom, der Rückführung des zurückgewonnenen Wasserstoffs zum ersten Synthesereaktor und ggf. der zeitlichen Variation des ersten und zweiten Spülstroms werden folgende Vorteile erhalten, die nachfolgend im Zusammenhang mit Fig. 1 beispielhaft erläutert werden:
In einem Beispiel wird das Verhältnis der Stoffmengenströme n1s/n2s des ersten Spülstroms n1s zum zweiten Spülstrom n2s zeitlich verändert. Auf diese Weise kann die Stöchiometriezahl für das zum ersten Synthesereaktor geführte Synthesegas jeweils auf den Zielwert von mindestens 2 eingestellt werden.

In einem weiteren Beispiel wird das Verhältnis der Stoffmengenströme n1 s/n2s des ersten Spülstroms n1s zum zweiten Spülstrom n2s zeitlich verändert und/oder die Summe des ersten Spülstroms und des zweiten Spülstroms n1s + n2s zeitlich verändert. Auf diese Weise kann auf die sich zeitlich verändernde Katalysatoraktivität in den mindestens zwei hintereinandergeschalteten Synthesereaktoren reagiert und die Stöchiometriezahl für das zum ersten Synthesereaktor geführte Synthesegas jeweils auf den Zielwert von mindestens 2 eingestellt werden.

In einem weiteren Beispiel wird das Verfahren während eines ersten Zeitabschnitts t1 mit einem Verhältnis der Stoffmengenströme (n1s/n2s)1 und während eines zweiten Zeitabschnitts t2 mit einem Verhältnis der Stoffmengenströme (n1s/n2s)2 durchgeführt, wobei die mittlere Katalysatoraktivität des Katalysators im ersten Methanolsynthesereaktor während des ersten Zeitabschnitts höher ist als während des zweiten Zeitabschnitts. Auf diese Weise kann auf die sich während der Zeitabschnitte t1 und t2 zeitlich verändernde Katalysatoraktivität in den mindestens zwei hintereinandergeschalteten Synthesereaktoren reagiert und die Stöchiometriezahl für das zum ersten Synthesereaktor geführte Synthesegas jeweils auf den Zielwert von mindestens 2 eingestellt werden, indem im ersten Zeitabschnitt t1 ein Verhältnis der Stoffmengenströme (n1s/n2s)1 und während eines zweiten Zeitabschnitts t2 ein Verhältnis der Stoffmengenströme (n1s/n2s)2 eingestellt wird. Die Aufteilung der Betriebsdauer der Syntheseanlage in zwei Zeitabschnitte ist dabei nur exemplarisch und nicht einschränkend zu verstehen. Es kann durchaus sinnvoll sein, weitere Zeitabschnitte t3, t4 usw. zu definieren, während derer weitere Stoffmengenstrom-Verhältnisse n1s/n2s)3, n1s/n2s)4 usw. eingestellt werden, um eine besonders kleinschrittige Einstellung der gewünschten Stöchiometriezahl zu ermöglichen. Dabei sollte aber der vergrößerte Regelaufwand in Bezug auf das verbesserte Einstellen der Stöchiometriezahl vertretbar bleiben.

In einem weiteren Beispiel wird das Verfahren während eines ersten Zeitabschnitts mit einem Verhältnis der Stoffmengenströme (n1s/n2s)1 und während eines ersten Zeitabschnitts mit einem Verhältnis der Stoffmengenströme (n1s/n2s)2 durchgeführt, wobei (n1s/n2s)1 größer ist als (n1s/n2s)2.

### Zahlenbeispiele

Es wurden Simulationsrechnungen für eine zweistufige Methanolsynthese mit wassergekühltem Reaktor (WCR, erster Synthesereaktor) und gasgekühltem Reaktor (GCR, zweiter Synthesereaktor) durchgeführt. Als Ausgangsmaterial diente dabei ein simuliertes Erdgas, enthaltend 1 Vol.-% C₂-Kohlenwasserstoffe, 1 Vol.-% Stickstoff, 1 Vol.-% Kohlendioxid, Rest Methan, das in Synthesegaserzeugungsstufe in Synthesegas umgewandelt und dann der Methanolsynthese zugeführt wurde.

Nach jeder Synthesestufe wurde das gebildete Methanolprodukt auskondensiert und es wurde aus dem jeweils verbleibenden Restgas ein Teil als Spülgas zu einer Wasserstoffrückgewinnungsvorrichtung geleitet, die als PSA-Anlage ausgestaltet war. Der nicht als Spülgas ausgeleitete Teil des jeweiligen Restgases wurde zur nachfolgenden Synthesestufe geführt bzw. zur ersten Synthesestufe zurückgeführt. Die Desaktivierung der Katalysatoren im ersten und zweiten Synthesereaktor wurde mittels eines mathematischen Modells simuliert, um die Unterschiede zwischen dem ersten Zeitabschnitt t1 (Start of Run, SOR) und dem zweiten Zeitabschnitt t2 (End of Run, EOR) abzubilden. Folgende Zielgrößen wurden dabei für SOR und EOR gewählt:
- Methanolproduktion jeweils rund 4020 Tagestonnen
- Stöchiometriezahl SN am WCR jeweils 2,9
- Wasserstoffverlust jeweils 1 % (als Brenngas)

Um diese Zielgrößen bei fortschreitender Katalysatordesaktivierung zu realisieren, war es erforderlich, bei SOR-Bedingungen (erster Zeitabschnitt) ein Verhältnis der Spülgas-Stoffmengenströme (n1s/n2s)1 von 2,6 und unter EOR-Bedingungen (zweiter Zeitabschnitt) ein Verhältnis der Spülgas-Stoffmengenströme (n1s/n2s)2 von 0,6 einzustellen.

### Bezugszeichenliste

- [1]: Verfahren, Anlage
- [10]: Leitung
- [11]: Verdichter
- [12]: Leitung
- [16]: Mischvorrichtung
- [17]: Leitung
- [18]: Leitung
- [19]: Leitung
- [20]: erster Methanolsynthesereaktor
- [22]: Leitung
- [23]: Wärmetauscher
- [24]: Leitung
- [25]: Kühler
- [26]: Leitung
- [30]: erste Phasentrennvorrichtung (Flüssigkeitsabscheider)
- [31]: Leitung
- [32]: Leitung
- [34]: Leitung
- [40]: zweiter Methanolsynthesereaktor
- [42]: Leitung
- [43]: Wärmetauscher
- [44]: Leitung
- [45]: Kühler
- [46]: Leitung
- [50]: zweite Phasentrennvorrichtung (Flüssigkeitsabscheider)
- [51]: Leitung
- [52]: Leitung
- [53]: Mischvorrichtung
- [54]: Leitung
- [55]: Leitung
- [56]: Verdichter
- [60]: Wasserstoffrückgewinnungsvorrichtung
- [61]: Leitung
- [62]: Regelventil
- [63]: Leitung
- [64]: Leitung
- [65]: Regelventil
- [66]: Leitung
- [67]: Leitung

## Patentansprüche

1. Verfahren zum Herstellen von Methanol aus einem Kohlenstoff enthaltenden Eisatzstrom, umfassend folgende Verfahrensschritte:
(a) Herstellen eines Wasserstoff und Kohlenoxide enthaltenden Synthesegaseinsatzstroms mittels eines Synthesegaserzeugungsverfahrens unter Synthesegaserzeugungsbedingungen aus dem Kohlenstoff enthaltenden Einsatzstrom,
(b) Zusammenführen und Vermischen des Synthesegaseinsatzstroms mit einem Wasserstoff und Kohlenoxide enthaltenden Recyclestrom zu einem ersten Reaktorfeedstrom,
(c) Einleiten des ersten Reaktorfeedstroms in einen ersten Methanolsynthesereaktor, enthaltend mindestens eine Katalysatorzone mit einem für die Methanolsynthese aktiven, festen, körnigen Katalysator, mindestens teilweises Umsetzen des ersten Reaktorfeedstroms in dem ersten Methanolsynthesereaktor unter Methanolsynthesebedingungen,
(d) Ausleiten eines Methanol und Wasser enthaltenden ersten Reaktorproduktstroms aus dem ersten Methanolsynthesereaktor, Abkühlen des ersten Reaktorproduktstroms unter seinen Taupunkt und Zuführen des abgekühlten ersten Reaktorproduktstroms zu einer ersten Phasentrennvorrichtung,
(e) Auftrennen des abgekühlten ersten Reaktorproduktstroms in der ersten Phasentrennvorrichtung in einen ersten Flüssigproduktstrom und einen ersten Restgasstrom, der nicht umgesetzte Synthesegasbestandteile enthält,
(f) Aufteilen des ersten Restgasstroms in einen zweiten Reaktorfeedstrom und in einen ersten Spülstrom,
(g) Einleiten des zweiten Reaktorfeedstroms in einen zweiten Methanolsynthesereaktor, enthaltend mindestens eine Katalysatorzone mit einem für die Methanolsynthese aktiven, festen, körnigen Katalysator, mindestens teilweises Umsetzen des zweiten Reaktorfeedstroms in dem zweiten Methanolsynthesereaktor unter Methanolsynthesebedingungen,
(h) Ausleiten eines Methanol und Wasser enthaltenden zweiten Reaktorproduktstroms aus dem zweiten Methanolsynthesereaktor, Abkühlen des zweiten Reaktorproduktstroms unter seinen Taupunkt und Zuführen des abgekühlten zweiten Reaktorproduktstroms zu einer zweiten Phasentrennvorrichtung,
(i) Auftrennen des abgekühlten zweiten Reaktorproduktstroms in der zweiten Phasentrennvorrichtung in einen zweiten Flüssigproduktstrom und einen zweiten Restgasstrom, der nicht umgesetzte Synthesegasbestandteile enthält,
(j) Aufteilen des zweiten Restgasstroms in den Recyclestrom, der zu Schritt (b) zurückgeführt wird, und in einen zweiten Spülstrom,
(k) Ausleiten des ersten und des zweiten Flüssigproduktstroms aus dem Verfahren als Rohmethanolproduktstrom,
(l) Einleiten mindestens eines Teils des ersten Spülstroms und mindestens eines Teils des zweiten Spülstroms in eine Wasserstoffrückgewinnungsvorrichtung, Auftrennen des ersten Spülstroms und des zweiten Spülstroms in der Wasserstoffrückgewinnungsvorrichtung in einen an Wasserstoff angereicherten Zusatzstrom und einen an Wasserstoff abgereicherten, dritten Spülstrom,
(m) Rückführen mindestens eines Teils des an Wasserstoff angereicherten Zusatzstroms zum ersten Methanolsynthesereaktor und Ausleiten des an Wasserstoff abgereicherten, dritten Spülstrom aus dem Verfahren.

2. Verfahren nach Anspruch 1, **dadurch gekennzeichnet, dass** der an Wasserstoff angereicherte Zusatzstrom vor dem Einleiten in den ersten Methanolsynthesereaktor mit dem Synthesegaseinsatzstrom und/oder mit dem Recyclestrom vermischt wird.

3. Verfahren nach Anspruch 1 oder 2, **dadurch gekennzeichnet, dass** der erste Spülstrom und der zweite Spülstrom in eine gemeinsame Wasserstoffrückgewinnungsvorrichtung eingeleitet werden.

4. Verfahren nach einem der vorigen Ansprüche, **dadurch gekennzeichnet, dass** die Wasserstoffrückgewinnungsvorrichtung nach dem Prinzip der Druckwechseladsorption (PSA) und/oder nach dem Prinzip der Membrantrennung arbeitet.

5. Verfahren nach einem der vorigen Ansprüche, **dadurch gekennzeichnet, dass** das Verhältnis der Stoffmengenströme n1s/n2s des ersten Spülstroms n1s zum zweiten Spülstrom n2s zeitlich verändert wird.

6. Verfahren nach einem der vorigen Ansprüche, **dadurch gekennzeichnet, dass** das Verhältnis der Stoffmengenströme n1s/n2s des ersten Spülstroms n1s zum zweiten Spülstrom n2s zeitlich verändert wird und/oder dass die Summe des ersten Spülstroms und des zweiten Spülstroms n1s + n2s zeitlich verändert wird.

7. Verfahren nach einem der vorigen Ansprüche, **dadurch gekennzeichnet, dass** das Verfahren während eines ersten Zeitabschnitts t1 mit einem Verhältnis der Stoffmengenströme (n1s/n2s)1 und während eines zweiten Zeitabschnitts t2 mit einem Verhältnis der Stoffmengenströme (n1s/n2s)2 durchgeführt wird, wobei die mittlere Katalysatoraktivität des Katalysators im ersten Methanolsynthesereaktor während des ersten Zeitabschnitts höher ist als während des zweiten Zeitabschnitts.

8. Verfahren nach einem der vorigen Ansprüche, **dadurch gekennzeichnet, dass** das Verfahren während eines ersten Zeitabschnitts t1 mit einem Verhältnis der Stoffmengenströme (n1s/n2s)1 und während eines zweiten Zeitabschnitts t2 mit einem Verhältnis der Stoffmengenströme (n1s/n2s)2 durchgeführt wird, wobei (n1s/n2s)1 größer ist als (n1s/n2s)2.

9. Verfahren nach Anspruch 8, **dadurch gekennzeichnet, dass** der erste Zeitabschnitt t1 mit der Inbetriebnahme des Verfahrens beginnt und dass der zweite Zeitabschnitt t2 mit der Außerbetriebnahme des Verfahrens endet.

10. Verfahren nach Anspruch 8 oder 9, **dadurch gekennzeichnet, dass** mit dem Beginn des ersten Zeitabschnitts t1 der Katalysatorzyklus in allen Methanolsynthesereaktoren beginnt (SOR) und dass mit dem Ende des zweiten Zeitabschnitts t2 der Katalysatorzyklus in allen Methanolsynthesereaktoren endet (EOR).

11. Verfahren nach einem der Ansprüche 8 bis 10, **dadurch gekennzeichnet, dass** das Verhältnis der Stoffmengenströme (n1s/n2s)1 größer als 1 ist und dass das Verhältnis der Stoffmengenströme (n1s/n2s)2 kleiner als 1 ist.

12. Verfahren nach einem der Ansprüche 8 bis 11, **dadurch gekennzeichnet, dass** das Verhältnis der Stoffmengenströme (n1s/n2s)1 zwischen 1 und 3, bevorzugt zwischen 2 und 2,9, meist bevorzugt zwischen 2,5 und 2,8 beträgt und dass das Verhältnis der Stoffmengenströme (n1s/n2s)2 zwischen 0 und 1, bevorzugt zwischen 0,3 und 0,9, meist bevorzugt zwischen 0,4 und 0,7 beträgt.

13. Verfahren nach einem der vorigen Ansprüche, **dadurch gekennzeichnet, dass** die zeitliche Abnahme des Mengenstroms des ersten Flüssigproduktstroms als Maß für das Abnehmen der Katalysatoraktivität des Katalysators im ersten Methanolsynthesereaktor dient.

14. Verfahren nach einem der vorigen Ansprüche, **dadurch gekennzeichnet, dass** mittels einer Temperaturmessvorrichtung wiederholt während der Durchführung des Verfahrens axiale Temperaturprofile innerhalb des Katalysatorbetts im ersten Methanolsynthesereaktor gemessen werden und dass die zeitliche Veränderung des axialen Temperaturprofils als Maß für das Abnehmen der Katalysatoraktivität des Katalysators im ersten Methanolsynthesereaktor dient.

15. Verfahren nach einem der vorigen Ansprüche, **dadurch gekennzeichnet, dass** das Synthesegaserzeugungsverfahren eine nichtkatalytische Partialoxidation (POX) und/oder eine autotherme Reformierung (ATR) umfasst.

16. Verfahren nach einem der vorigen Ansprüche, **dadurch gekennzeichnet, dass** die Stöchiometriezahl des Synthesegaseinsatzstroms unter 2 beträgt und dass die Stöchiometriezahl des aus dem Synthesegaseinsatzstrom, dem Recyclestrom und dem Zusatzstrom erhaltenen Gasgemischs über 2 beträgt, bevor dieses in den ersten Methanolsynthesereaktor eingeleitet wird.

17. Anlage zum Herstellen von Methanol aus einem Kohlenstoff enthaltenden Eisatzstrom, umfassend folgende, miteinander in Fluidverbindung stehende Bestandteile:
(a) Eine nach einem Synthesegaserzeugungsverfahren arbeitende Synthesegaserzeugungsanlage, geeignet zum Herstellen eines Wasserstoff und Kohlenoxide enthaltenden Synthesegaseinsatzstroms aus dem Kohlenstoff enthaltenden Einsatzstrom,
(b) Mittel zum Zusammenführen und Vermischen des Synthesegaseinsatzstroms mit einem Wasserstoff und Kohlenoxide enthaltenden Recyclestrom zu einem ersten Reaktorfeedstrom,
(c) einen ersten Methanolsynthesereaktor, enthaltend mindestens eine Katalysatorzone mit einem für die Methanolsynthese aktiven, festen, körnigen Katalysator, Mittel zum Einleiten des ersten Reaktorfeedstroms in den ersten Methanolsynthesereaktor,
(d) Mittel zum Ausleiten eines Methanol und Wasser enthaltenden ersten Reaktorproduktstroms aus dem ersten Methanolsynthesereaktor, Mittel zum Abkühlen des ersten Reaktorproduktstroms unter seinen Taupunkt, eine erste Phasentrennvorrichtung, Mittel zum Zuführen des abgekühlten ersten Reaktorproduktstroms zu der ersten Phasentrennvorrichtung,
(e) Mittel zum Ausleiten eines ersten Flüssigproduktstroms und einen ersten Restgasstroms, der nicht umgesetzte Synthesegasbestandteile enthält, aus der ersten Phasentrennvorrichtung,
(f) Mittel zum Aufteilen des ersten Restgasstroms in einen zweiten Reaktorfeedstrom und in einen ersten Spülstrom,
(g) einen zweiten Methanolsynthesereaktor, enthaltend mindestens eine Katalysatorzone mit einem für die Methanolsynthese aktiven, festen, körnigen Katalysator, Mittel zum Einleiten des zweiten Reaktorfeedstroms in den zweiten Methanolsynthesereaktor,
(h) Mittel zum Ausleiten eines Methanol und Wasser enthaltenden zweiten Reaktorproduktstroms aus dem zweiten Methanolsynthesereaktor, Mittel zum Abkühlen des zweiten Reaktorproduktstroms unter seinen Taupunkt, eine zweite Phasentrennvorrichtung, Mittel zum Zuführen des abgekühlten zweiten Reaktorproduktstroms zu der zweiten Phasentrennvorrichtung,
(i) Mittel zum Ausleiten eines zweiten Flüssigproduktstroms und eines zweiten Restgasstroms, der nicht umgesetzte Synthesegasbestandteile enthält, aus der zweiten Phasentrennvorrichtung,
(j) Mittel zum Aufteilen des zweiten Restgasstroms in den Recyclestrom und in einen zweiten Spülstrom, Mittel zum Rückführen des Recyclestroms zu Bestandteil (b),
(k) Mittel zum Ausleiten des ersten und des zweiten Flüssigproduktstroms aus der Anlage als Rohmethanolproduktstrom,
(l) mindestens eine Wasserstoffrückgewinnungsvorrichtung, Mittel zum Einleiten des ersten Spülstroms und des zweiten Spülstroms in die mindestens eine Wasserstoffrückgewinnungsvorrichtung, wobei die Mittel so beschaffen sind, dass der erste Spülstrom und/oder der zweite Spülstrom und/oder die Summe beider Spülströme zeitlich veränderbar ist, Mittel zum Ausleiten eines an Wasserstoff angereicherten Zusatzstroms und eines an Wasserstoff abgereicherten, dritten Spülstroms aus der Wasserstoffrückgewinnungsvorrichtung,
(m) Mittel zum Rückführen mindestens eines Teils des an Wasserstoff angereicherten Zusatzstroms zum ersten Methanolsynthesereaktor und Mittel zum Ausleiten des an Wasserstoff abgereicherten, dritten Spülstroms aus dem Verfahren.

## Claims

1. Process for producing methanol from a carbon-containing input stream comprising the following process steps:
(a) producing a synthesis gas input stream containing hydrogen and carbon oxides using a synthesis gas production process under synthesis gas production conditions from the carbon-containing input stream,
(b) combining and mixing the synthesis gas input stream with a recycle stream containing hydrogen and carbon oxides to afford a first reactor feed stream,
(c) introducing the first reactor feed stream into a first methanol synthesis reactor containing at least one catalyst zone comprising a solid, granular catalyst active for methanol synthesis, at least partially converting the first reactor feed stream in the first methanol synthesis reactor under methanol synthesis conditions,
(d) discharging a first reactor product stream containing methanol and water from the first methanol synthesis reactor, cooling the first reactor product stream below its dew point and supplying the cooled first reactor product stream to a first phase separation apparatus,
(e) separating the cooled first reactor product stream in the first phase separation apparatus into a first liquid product stream and a first residual gas stream containing unconverted synthesis gas constituents,
(f) dividing the first residual gas stream into a second reactor feed stream and into a first purge stream,
(g) introducing the second reactor feed stream into a second methanol synthesis reactor containing at least one catalyst zone comprising a solid, granular catalyst active for methanol synthesis, at least partially converting the second reactor feed stream in the second methanol synthesis reactor under methanol synthesis conditions,
(h) discharging a second reactor product stream containing methanol and water from the second methanol synthesis reactor, cooling the second reactor product stream below its dew point and supplying the cooled second reactor product stream to a second phase separation apparatus,
(i) separating the cooled second reactor product stream in the second phase separation apparatus into a second liquid product stream and a second residual gas stream containing unconverted synthesis gas constituents,
(j) dividing the second residual gas stream into the recycle stream recycled to step (b) and into a second purge stream,
(k) discharging the first and the second liquid product stream from the process as a crude methanol product stream,
(l) introducing at least a portion of the first purge stream and at least a portion of the second purge stream into a hydrogen recovery apparatus, separating the first purge stream and the second purge stream in the hydrogen recovery apparatus into a hydrogen-enriched supplementary stream and a hydrogen-depleted third purge stream,
(m) recycling at least a portion of the hydrogen-enriched supplementary stream to the first methanol synthesis reactor and discharging the hydrogen-depleted third purge stream from the process.

2. Process according to Claim 1, **characterized in that** the hydrogen-enriched supplementary stream is mixed with the synthesis gas input stream and/or with the recycle stream before introduction into the first methanol synthesis reactor.

3. Process according to Claim 1 or 2, **characterized in that** the first purge stream and the second purge stream are introduced into a common hydrogen recovery apparatus.

4. Process according to any of the preceding claims, **characterized in that** the hydrogen recovery apparatus operates according to the principle of pressure swing adsorption (PSA) and/or according to the principle of membrane separation.

5. Process according to any of the preceding claims, **characterized in that** the ratio of the molar flows nls/n2s of the first purge stream n1s to the second purge stream n2s is altered over time.

6. Process according to any of the preceding claims, **characterized in that** the ratio of the molar flows nls/n2s of the first purge stream n1s to the second purge stream n2s is altered over time and/or **in that** the sum of the first purge stream and of the second purge stream ols + n2s is altered over time.

7. Process according to any of the preceding claims, **characterized in that** the process is performed at a ratio of the molar flows (nls/n2s)1 during a first time interval t1 and at a ratio of the molar flows (nls/n2s)2 during a second time interval t2, wherein the average catalyst activity of the catalyst in the first methanol synthesis reactor is higher during the first time interval than during the second time interval.

8. Process according to any of the preceding claims, **characterized in that** the process is performed at a ratio of the molar flows (nls/n2s)1 during a first time interval t1 and at a ratio of the molar flows (nls/n2s)2 during a second time interval t2, wherein (nls/n2s)1 is greater than (nls/n2s)2.

9. Process according to Claim 8, **characterized in that** the first time interval t1 begins with the startup of the process and **in that** the second time interval t2 ends with the shutdown of the process.

10. Process according to Claim 8 or 9, **characterized in that** the beginning of the first time interval t1 corresponds to the beginning of the catalyst cycle in all methanol synthesis reactors (SOR) and **in that** the end of the second time interval t2 corresponds to the end of the catalyst cycle in all methanol synthesis reactors (EOR).

11. Process according to any of Claims 8 to 10, **characterized in that** the ratio of the molar flows (nls/n2s)1 is greater than 1 and **in that** the ratio of the molar flows (nls/n2s)2 is less than 1.

12. Process according to any of Claims 8 to 11, **characterized in that** the ratio of the molar flows (n1s/n2s)1 is between 1 and 3, preferably between 2 and 2.9, most preferably between 2.5 and 2.8, and **in that** the ratio of the molar flows (nls/n2s)2 is between 0 and 1, preferably between 0.3 and 0.9, most preferably between 0.4 and 0.7.

13. Process according to any of the preceding claims, **characterized in that** the reduction in the molar flow of the first liquid product stream over time serves as a measure for the reduction in catalyst activity of the catalyst in the first methanol synthesis reactor.

14. Process according to any of the preceding claims, **characterized in that** a temperature measurement apparatus is used to repeatedly measure during performance of the process axial temperature profiles within the catalyst bed in the first methanol synthesis reactor and **in that** the change in the axial temperature profile over time serves as a measure for the decrease in catalyst activity of the catalyst in the first methanol synthesis reactor.

15. Process according to any of the preceding claims, **characterized in that** the synthesis gas production process comprises a noncatalytic partial oxidation (POX) and/or an autothermal reforming (ATR).

16. Process according to any of the preceding claims, **characterized in that** the stoichiometry number of the synthesis gas input stream is less than 2 and **in that** the stoichiometry number of the gas mixture obtained from the synthesis gas input stream, the recycle stream and the supplementary stream is more than 2 before introduction thereof into the first methanol synthesis reactor.

17. Plant for producing methanol from a carbon-containing input stream, comprising the following constituents in fluid connection with one another:
(a) a synthesis gas production plant operating according to a synthesis gas production process suitable for producing a synthesis gas input stream containing hydrogen and carbon oxides from the carbon-containing input stream,
(b) means for combining and mixing the synthesis gas input stream with a recycle stream containing hydrogen and carbon oxides to afford a first reactor feed stream,
(c) a first methanol synthesis reactor containing at least one catalyst zone comprising a solid, granular catalyst active for methanol synthesis, means for introducing the first reactor feed stream into the first methanol synthesis reactor,
(d) means for discharging a first reactor product stream containing methanol and water from the first methanol synthesis reactor, means for cooling the first reactor product stream below its dew point, a first phase separation apparatus, means for supplying the cooled first reactor product stream to the first phase separation apparatus,
(e) means for discharging a first liquid product stream and a first residual gas stream containing unconverted synthesis gas constituents from the first phase separation apparatus,
(f) means for dividing the first residual gas stream into a second reactor feed stream and into a first purge stream,
(g) a second methanol synthesis reactor containing at least one catalyst zone comprising a solid, granular catalyst active for methanol synthesis, means for introducing the second reactor feed stream into the second methanol synthesis reactor,
(h) means for discharging a second reactor product stream containing methanol and water from the second methanol synthesis reactor, means for cooling the second reactor product stream below its dew point, a second phase separation apparatus, means for supplying the cooled second reactor product stream to the second phase separation apparatus,
(i) means for discharging a second liquid product stream and a second residual gas stream containing unconverted synthesis gas constituents from the second phase separation apparatus,
(j) means for: dividing the second residual gas stream into the recycle stream and into a second purge stream, means for recycling the recycle stream to constituent (b),
(k) means for discharging the first and the second liquid product stream from the plant as a crude methanol product stream,
(l) at least one hydrogen recovery apparatus, means for introducing the first purge stream and the second purge stream into the at least one hydrogen recovery apparatus, wherein the means are constituted such that the first purge stream and/or the second purge stream and/or the sum of both purge streams are alterable over time, means for discharging a hydrogen-enriched supplementary stream and a hydrogen-depleted third purge stream from the hydrogen recovery apparatus,
(m) means for recycling at least a portion of the hydrogen-enriched supplementary stream to the first methanol synthesis reactor and means for discharging the hydrogen-depleted third purge stream from the process.

## Revendications

1. Procédé de préparation de méthanol à partir d'un courant d'entrée contenant du carbone, comprenant les étapes de procédé suivantes :
(a) la préparation d'un courant d'entrée de gaz de synthèse contenant de l'hydrogène et des oxydes de carbone au moyen d'un procédé de production de gaz de synthèse dans des conditions de production de gaz de synthèse à partir du courant d'entrée contenant du carbone,
(b) la réunion et le mélange du courant d'entrée de gaz de synthèse avec un courant de recyclage contenant de l'hydrogène et des oxydes de carbone pour former un premier courant d'alimentation de réacteur,
(c) l'introduction du premier courant d'alimentation de réacteur dans un premier réacteur de synthèse de méthanol, contenant au moins une zone de catalyseur avec un catalyseur granulaire solide actif pour la synthèse de méthanol, la mise en réaction au moins partielle du premier courant d'alimentation de réacteur dans le premier réacteur de synthèse du méthanol dans des conditions de synthèse de méthanol,
(d) le déchargement d'un premier courant de produit de réacteur contenant du méthanol et de l'eau du premier réacteur de synthèse de méthanol, le refroidissement du premier courant de produit de réacteur en dessous de son point de rosée et l'acheminement du premier courant de produit de réacteur refroidi vers un premier dispositif de séparation de phases,
(e) la séparation du premier courant de produit de réacteur refroidi dans le premier dispositif de séparation de phases en un premier courant de produit liquide et un premier courant de gaz résiduel, qui contient des constituants du gaz de synthèse n'ayant pas réagi,
(f) la division du premier courant de gaz résiduel en un deuxième courant d'alimentation de réacteur et en un premier courant de rinçage,
(g) l'introduction du deuxième courant d'alimentation de réacteur dans un deuxième réacteur de synthèse de méthanol, contenant au moins une zone de catalyseur avec un catalyseur granulaire solide actif pour la synthèse de méthanol, la mise en réaction au moins partielle du deuxième courant d'alimentation de réacteur dans le deuxième réacteur de synthèse de méthanol dans des conditions de synthèse de méthanol,
(h) le déchargement d'un deuxième courant de produit de réacteur contenant du méthanol et de l'eau du deuxième réacteur de synthèse de méthanol, le refroidissement du deuxième courant de produit de réacteur en dessous de son point de rosée et l'acheminement du deuxième courant de produit de réacteur refroidi vers un deuxième dispositif de séparation de phases,
(i) la séparation du deuxième courant de produit de réacteur refroidi dans le deuxième dispositif de séparation de phases en un deuxième courant de produit liquide et un deuxième courant de gaz résiduel, qui contient des constituants du gaz de synthèse n'ayant pas réagi,
(j) la division du deuxième courant de gaz résiduel en le courant de recyclage, qui est renvoyé vers l'étape (b), et en un deuxième courant de rinçage,
(k) le déchargement du le premier et du deuxième courant de produit liquide du procédé en tant que courant de produit de méthanol brut,
(l) l'introduction d'au moins une partie du premier courant de rinçage et d'au moins une partie du deuxième courant de rinçage dans un dispositif de récupération d'hydrogène, la séparation du premier courant de rinçage et du deuxième courant de rinçage dans le dispositif de récupération d'hydrogène en un courant supplémentaire enrichi en hydrogène et un troisième courant de rinçage appauvri en hydrogène,
(m) le renvoi d'au moins une partie du courant supplémentaire enrichi en hydrogène vers le premier réacteur de synthèse de méthanol et le déchargement du troisième courant de rinçage appauvri en hydrogène du procédé.

2. Procédé selon la revendication 1, **caractérisé en ce que** le courant supplémentaire enrichi en hydrogène est mélangé avec le courant d'entrée de gaz de synthèse et/ou avec le courant de recyclage avant l'introduction dans le premier réacteur de synthèse de méthanol,

3. Procédé selon la revendication 1 ou 2, **caractérisé en ce que** le premier courant de rinçage et le deuxième courant de rinçage sont introduits dans un dispositif de récupération d'hydrogène commun.

4. Procédé selon l'une quelconque des revendications précédentes, **caractérisé en ce que** le dispositif de récupération d'hydrogène fonctionne selon le principe de l'adsorption modulée en pression (PSA) et/ou selon le principe de la séparation par membrane.

5. Procédé selon l'une quelconque des revendications précédentes, **caractérisé en ce que** le rapport des débits de matière n1s/n2s du premier courant de rinçage n1s au deuxième courant de rinçage n2s est varié dans le temps.

6. Procédé selon l'une quelconque des revendications précédentes, **caractérisé en ce que** le rapport des débits de matière nls/n2s du premier courant de rinçage nls au deuxième courant de rinçage n2s est varié dans le temps et/ou **en ce que** la somme du premier courant de rinçage et du deuxième courant de rinçage n1s+n2s est variée dans le temps.

7. Procédé selon l'une quelconque des revendications précédentes, **caractérisé en ce que** le procédé est réalisé pendant une première période de temps t1 avec un rapport des débits de matière (n1s/n2s)1 et pendant une deuxième période de temps t2 avec un rapport des débits de matière (n1s/n2s)2, l'activité catalytique moyenne du catalyseur dans le premier réacteur de synthèse de méthanol étant plus élevée pendant la première période de temps que pendant la deuxième période de temps.

8. Procédé selon l'une quelconque des revendications précédentes, **caractérisé en ce que** le procédé est réalisé pendant une première période de temps t1 avec un rapport des débits de matière (n1s/n2s)1 et pendant une deuxième période de temps t2 avec un rapport des débits de matière (n1s/n2s)2, (n1s/n2s)1 étant supérieur à (n1s/n2s)2.

9. Procédé selon la revendication 8, **caractérisé en ce que** la première période de temps t1 commence avec la mise en service du procédé et **en ce que** la deuxième période de temps t2 se termine avec la mise hors service du procédé.

10. Procédé selon la revendication 8 ou 9, **caractérisé en ce qu'**au début de la première période de temps t1, le cycle du catalyseur commence dans tous les réacteurs de synthèse de méthanol (SOR) et **en ce qu'**à la fin de la deuxième période de temps t2, le cycle du catalyseur se termine dans tous les réacteurs de synthèse de méthanol (PCP) .

11. Procédé selon l'une quelconque des revendications 8 à 10, **caractérisé en ce que** le rapport des débits de matière (nls/n2s)1 est supérieur à 1 et **en ce que** le rapport des débits de matière (n1s/n2s)2 est inférieur à 1.

12. Procédé selon l'une quelconque des revendications 8 à 11, **caractérisé en ce que** le rapport des débits de matière (n1s/n2s)1 est compris entre 1 et 3, de préférence entre 2 et 2,9, plus préférablement entre 2,5 et 2,8, et **en ce que** le rapport des débits de matière (nls/n2s)2 est compris entre 0 et 1, de préférence entre 0,3 et 0,9, plus préférablement entre 0,4 et 0,7.

13. Procédé selon l'une quelconque des revendications précédentes, **caractérisé en ce que** la diminution dans le temps du débit du premier courant de produit liquide sert de mesure de la diminution de l'activité catalytique du catalyseur dans le premier réacteur de synthèse de méthanol.

14. Procédé selon l'une quelconque des revendications précédentes, **caractérisé en ce que** des profils de température axiaux sont mesurés de manière répétée à l'intérieur du lit de catalyseur dans le premier réacteur de synthèse de méthanol au moyen d'un dispositif de mesure de la température pendant la réalisation du procédé et **en ce que** la variation dans le temps du profil de température axial sert de mesure de la diminution de l'activité catalytique du catalyseur dans le premier réacteur de synthèse de méthanol.

15. Procédé selon l'une quelconque des revendications précédentes, **caractérisé en ce que** le procédé de production de gaz de synthèse comprend une oxydation partielle non catalytique (POX) et/ou un reformage autothermique (ATR).

16. Procédé selon l'une quelconque des revendications précédentes, **caractérisé en ce que** l'indice stoechiométrique du courant d'entrée de gaz de synthèse est inférieur à 2 et **en ce que** l'indice stoechiométrique du mélange de gaz obtenu à partir du courant d'entrée de gaz de synthèse, du courant de recyclage et du courant supplémentaire est supérieur à 2 avant que celui-ci soit introduit dans le premier réacteur de synthèse de méthanol.

17. Installation pour préparer du méthanol à partir d'un courant d'entrée contenant du carbone, comprenant les constituants suivants en communication fluidique les uns avec les autres :
(a) une installation de production de gaz de synthèse fonctionnant selon un procédé de production de gaz de synthèse, appropriée pour préparer un courant d'entrée de gaz de synthèse contenant de l'hydrogène et des oxydes de carbone à partir du courant d'entrée contenant du carbone,
(b) des moyens pour réunir et mélanger le courant d'entrée de gaz de synthèse avec un courant de recyclage contenant de l'hydrogène et des oxydes de carbone pour former un premier courant d'alimentation de réacteur,
(c) un premier réacteur de synthèse de méthanol, contenant au moins une zone de catalyseur avec un catalyseur granulaire solide actif pour la synthèse de méthanol, des moyens pour introduire le premier courant d'alimentation de réacteur dans le premier réacteur de synthèse de méthanol,
(d) des moyens pour décharger un premier courant de produit de réacteur contenant du méthanol et de l'eau du premier réacteur de synthèse de méthanol, des moyens pour refroidir le premier courant de produit de réacteur en dessous de son point de rosée, un premier dispositif de séparation de phases, des moyens pour acheminer le premier courant de produit de réacteur refroidi vers le premier dispositif de séparation de phases,
(e) des moyens pour décharger un premier courant de produit liquide et un premier courant de gaz résiduel, qui contient des constituants du gaz de synthèse n'ayant pas réagi, du premier dispositif de séparation de phases,
(f) des moyens pour diviser le premier courant de gaz résiduel en un deuxième courant d'alimentation de réacteur et en un premier courant de rinçage,
(g) un deuxième réacteur de synthèse de méthanol, contenant au moins une zone de catalyseur avec un catalyseur granulaire solide actif pour la synthèse de méthanol, des moyens pour introduire le deuxième courant d'alimentation de réacteur dans le deuxième réacteur de synthèse de méthanol,
(h) des moyens pour décharger un deuxième courant de produit de réacteur contenant du méthanol et de l'eau du deuxième réacteur de synthèse de méthanol, des moyens pour refroidir le deuxième courant de produit de réacteur en dessous de son point de rosée, un deuxième dispositif de séparation de phases, des moyens pour acheminer le deuxième courant de produit de réacteur refroidi vers le deuxième dispositif de séparation de phases,
(i) des moyens pour décharger un deuxième courant de produit liquide et un deuxième courant de gaz résiduel, qui contient des constituants du gaz de synthèse n'ayant pas réagi, du deuxième dispositif de séparation de phases,
(j) des moyens pour diviser le deuxième courant de gaz résiduel en le courant de recyclage et en un deuxième courant de rinçage, des moyens pour renvoyer le courant de recyclage vers le constituant (b),
(k) des moyens pour décharger le premier et le deuxième courant de produit liquide de l'installation en tant que courant de produit de méthanol brut,
(l) au moins un dispositif de récupération d'hydrogène, des moyens pour introduire le premier courant de rinçage et le deuxième courant de rinçage dans l'au moins un dispositif de récupération d'hydrogène, les moyens étant tels que le premier courant de rinçage et/ou le deuxième courant de rinçage et/ou la somme des deux courants de rinçage sont variables dans le temps, des moyens pour décharger un courant supplémentaire enrichi en hydrogène et un troisième courant de rinçage appauvri en hydrogène du dispositif de récupération d'hydrogène,
(m) des moyens pour renvoyer au moins une partie du courant supplémentaire enrichi en hydrogène vers le premier réacteur de synthèse de méthanol et des moyens pour décharger le troisième courant de rinçage appauvri en hydrogène du procédé.
